# EUROPEAN PATENT SPECIFICATION

(11) **EP 3 091 083 B1**
(45) Date of publication and mention of the grant of the patent: **08.11.2017**
(21) Application number: 15166675.7
(22) Date of filing: 07.05.2015
(51) Int. Cl.: C12Q 1/68

(54) **A KIT FOR DETECTING MUTATION OR POLYMORPHISM IN THE HUMAN MITOCHONDRIAL DNA**
KIT ZUM NACHWEIS VON MUTATION ODER VON POLYMORPHISMUS IN MITOCHONDRIALER DNA
KIT DE DÉTECTION D'UNE MUTATION OU D'UN POLYMORPHISME DANS L'ADN MITOCHONDRIAL HUMAIN

(43) Date of publication of application: 09.11.2016
(73) Proprietor: Latvian Biomedical Research and Study Centre, 1067 Riga (LV)
(72) Inventor: JANKEVICS, Eriks, deceased (LV); INASKINA, Inna, 1067 Riga (LV); PELNENA, Dita, 1067 Riga (LV); STAVUSIS, Janis, 1067 Riga (LV); PLISS, Liana, 1067 Riga (LV)
(74) Representative: Kuzjukevica, Lucija

(56) References cited:
- WO-A1-02/22873
- WO-A1-96/06187
- WO-A1-03/046225
- WO-A2-02/068629
- WO-A2-03/018775
- US-A- 789 556
- US-A1- 2006 078 881
- US-A1- 2013 274 314
- DATABASE Geneseq [Online] 22 August 2003 (2003-08-22), "Homo sapiens mitochondrion, complete genome", XP002747771, retrieved from EBI accession no. GSN:AF346985 Database accession no. AF346985
- WALLACE ET AL: "Mitochondrial diseases: genotype versus phenotype", TRENDS IN GENETICS, ELSEVIER SCIENCE PUBLISHERS B.V. AMSTERDAM, NL, vol. 9, no. 4, 1 April 1993 (1993-04-01), pages 128-133, XP023478603, ISSN: 0168-9525, DOI: 10.1016/0168-9525(93)90207-X [retrieved on 1993-04-01]
- AMANDA RAMOS ET AL: "Human mitochondrial DNA complete amplification and sequencing: A new validated primer set that prevents nuclear DNA sequences of mitochondrial origin co-amplification", ELECTROPHORESIS, vol. 30, no. 9, 1 May 2009 (2009-05-01), pages 1587-1593, XP55028394, ISSN: 0173-0835, DOI: 10.1002/elps.200800601
- TUPPEN H A L ET AL: "Mitochondrial DNA mutations and human disease", BIOCHIMICA ET BIOPHYSICA ACTA. BIOENERGETICS, AMSTERDAM, NL, vol. 1797, no. 2, 1 February 2010 (2010-02-01), pages 113-128, XP026855170, ISSN: 0005-2728 [retrieved on 2009-09-15]
- R. W. TAYLOR ET AL: "The determination of complete human mitochondrial DNA sequences in single cells: implications for the study of somatic mitochondrial DNA point mutations", NUCLEIC ACIDS RESEARCH, vol. 29, no. 15, 1 August 2001 (2001-08-01), pages 74e-74, XP055222695, DOI: 10.1093/nar/29.15.e74
- ZHOU SHAOYU ET AL: "An oligonucleotide microarray for high-throughput sequencing of the mitochondrial genome", JOURNAL OF MOLECULAR DIAGNOSTICS,THE, AMERICAN SOCIETY FOR INVESTIGATIVE PATHOLOGY, US, vol. 8, no. 4, 1 September 2006 (2006-09-01), pages 476-482, XP002461366, ISSN: 1525-1578, DOI: 10.2353/JMOLDX.2006.060008

## Description

### Field of the invention

The present invention relates to a kit for detecting mutation or polymorphism in the human mitochondrial DNA (mtDNA).

### Description of the related prior art

Mitochondria are dynamic cell structures that arose through endosymbiosis more than 1.45 billion years ago, preserving their circular DNA molecules (mtDNA), ribosomes, and 13 proteins (John, P. & Whatley, F. R. Paracoccus denitrificans and the evolutionary origin of the mitochondrion. Nature 254, 495-8 (1975)). They have a number of functions in the cell, like ATP synthesis, fatty acid oxidation, heme biosynthesis, heat generation, calcium homeostasis, citric acid cycle, and apoptosis (Edinger, A. L. & Thompson, C. B. Death by design: apoptosis, necrosis and autophagy. Curr. Opin. Cell Biol. 16, 663-9 (2004), McBride, H. M., Neuspiel, M. & Wasiak, S. Mitochondria: more than just a powerhouse. Curr. Biol. 16, R551-60 (2006), Lax, N. Z., Turnbull, D. M. & Reeve, A. K. Mitochondrial mutations: newly discovered players in neuronal degeneration. Neuroscientist 17, 645-58 (2011), Koopman, W. J. H., Willems, P. H. G. M. & Smeitink, J. A. M. Monogenic mitochondrial disorders. N. Engl. J. Med. 366, 1132-41 (2012)). Defects in the oxidative phosphorylation (OXPHOS) system cause mitochondrial diseases with various clinical manifestations, presenting at any age with non-specific clinical symptoms that are highly variable within families. Underlying mechanisms of the clinical heterogeneity could include heteroplasmy (i.e., DNA molecules with and without mutations within a single cell) and its occurrence predominant in tissues with high ATP demand (Wallace, D. C. Mitochondrial diseases: genotype versus phenotype. Trends Genet. 9, 128-33 (1993)). Molecular diagnostics of mitochondrial diseases is complicated by heteroplasmy, which varies between tissues, and by nuclear genes that have functions related to the synthesis of mtDNA-encoded proteins, enzymes involved in mitochondrial metabolism, oxidative phosphorylation assembly proteins, and more (Smeitink, J. A., Sengers, R. C., Trijbels, F. J. & van den Heuvel, L. P. Nuclear genes and oxidative phosphorylation disorders: a review. Eur. J. Pediatr. 159 Suppl, S227-31 (2000)).

Methods for biochemical and genetic diagnosis of mitochondrial diseases have advanced considerably over the past 50 years, since the first patient was reported in 1962 (Luft, R., Ikkos, D., Palmieri, G., Ernster, L. & Afzelius, B. A case of severe hypermetabolism of nonthyroid origin with a defect in the maintenance of mitochondrial respiratory control: a correlated clinical, biochemical, and morphological study. J. Clin. Invest. 41, 1776-804 (1962)). Mitochondrial dysfunctions are classified as primary (mutations in mtDNA or nuclear DNA-encoded mitochondrial genes) or secondary (outside influence on mitochondria, e.g., viral infections). Aging is also related to mitochondrial dysfunction. Heteroplasmy of mtDNA tends to increase with age owing to the high mtDNA mutation rate, which also contributes to the accumulation of somatic mtDNA mutations with age (Wallace, D. C. Mitochondrial DNA mutations in disease and aging. Environ. Mol. Mutagen. 51, 440-50 (2010)). One hypothesis suggests that some of these mutations are inherited, but at a low level and without clinical consequences (Payne, B. A. I. et al. Universal heteroplasmy of human mitochondrial DNA. Hum. Mol. Genet. 22, 384-90 (2013)).

Patients with primary mitochondrial disease usually suffer from multisystem organ symptoms, particularly those organs with high ATP demand, such as the central nervous system, muscle, heart muscle, vision, liver, and gastrointestinal and endocrine systems. Symptoms of the disease are not specific and contribute to a large differential diagnosis list; patients may present with "any symptom in any organ at any age" (Munnich, A. et al. Clinical presentation of mitochondrial disorders in childhood. J. Inherit. Metab. Dis. 19, 521-7 (1996)). There are several baseline screening tests for primary mitochondrial disease. They include pre- and postprandial capillary lactate measurements, the oral glucose tolerance test, urinary ketone measurements, serum amino acid analysis, and urine organic acid analysis. Additionally, lactate measurements in the cerebrospinal fluid and proton magnetic resonance spectroscopy can be used (Haas, R. H. et al. Mitochondrial disease: a practical approach for primary care physicians. Pediatrics 120, 1326-33 (2007); Haas, R. H. et al. The in-depth evaluation of suspected mitochondrial disease. Mol. Genet. Metab. 94, 16-37 (2008)). These investigations are limited because negative results do not exclude the possibility of mitochondrial disease. The Nijmegen Center for Mitochondrial Disorders developed and utilizes mitochondrial disease criteria, which allow combinations of various symptoms and tests, to reach a suspicion threshold indicating the need to proceed with further studies (Morava, E. et al. Mitochondrial disease criteria: diagnostic applications in children. Neurology 67, 1823-6 (2006)). Muscle biopsy with subsequent analyses of ATP production and enzymes is considered to be the gold standard in the diagnosis of mitochondrial pathology (DiMauro, S. & Schon, E. A. Mitochondrial respiratory-chain diseases. N. Engl. J. Med. 348, 2656-68 (2003)). It is a highly informative and valuable method for diagnosis, but has limitations. Enzyme analysis of the mitochondrial respiratory chain complex is performed only in a small number of highly specialized centers, and requires fresh muscle specimens properly collected from the correct tissue. This makes the test difficult to perform in all patients with suspected mitochondrial disease. Another pitfall is that results obtained from the analysis do not differentiate between primary or secondary causes of mitochondrial dysfunction (e.g., toxic drugs, malnutrition, and toxic metabolites) and require further diagnostic and research arrays (Rodenburg, R. J. T. Biochemical diagnosis of mitochondrial disorders. J. Inherit. Metab. Dis. 34, 283-92 (2011); Rahman, S. & Hanna, M. G. Diagnosis and therapy in neuromuscular disorders: diagnosis and new treatments in mitochondrial diseases. J. Neurol. Neurosurg. Psychiatry 80, 943-53 (2009); Koopman, W. J. H., Willems, P. H. G. M. & Smeitink, J. A. M. Monogenic mitochondrial disorders. N. Engl. J. Med. 366, 1132-41 (2012)).

US patent application No. US2006/0078881 discloses a method and kit for defection of mutations in human mitochondrial DNA sequences and specifically to the use of mitochondrial DNA variants (polymorphisms) with high mutation frequency to be employed in the comparison of biological samples with samples of known origin in the purpose of, for example, human identification or forensic genetics.

US patent No. US 8,008,008 discloses a method and kits for detecting mitochondrial DNA deletions for the early detection, diagnosis and progression of prostate cancer, sun exposure and non-melanoma skin cancer.

PCT application WO 02/22873 discloses a method and kit for determining the geographic or population origin of a human based on mitochondrial DNA sequences. The described kit allows simultaneously determination of various single nucleotide polymorphisms in human mtDNA.

The aim of the invention is to develop a robust, low-cost mutation screening kit for diagnostics of mitochondrial diseases.

### Summary of the invention

The present invention provides a test kit for detecting mutation or polymorphism in the human mitochondrial DNA. Said kit comprises probes comprising of SEQ ID Nos. 1 to 96 (see table 1 and 3).

The kit is useful for detecting such a mitochondrial diseases as chronic progressive external ophthalmoplegia (CPEO), Kearns-Sayre syndrome (KSS), Leber hereditary optic neuropathy (LHON), mitochondrial encephalomyopathy with lactic acidosis and stroke-like episodes (MELAS), myoclonic epilepsy myopathy sensory ataxia (MEMSA), myoclonic epilepsy with ragged-red fibres (MERRF), mitochondrial recessive ataxia syndrome (MIRAS), neurogenic weakness with ataxia and retinitis pigmentosa (NARP), sensory ataxia neuropathy, dysarthria, ophthalmoplegia (SANDO), spinocerebellar ataxia with epilepsy (SCAE), Alpers-Huttenlocher syndrome, Pearson syndrome, Infantile myopathy and lactic acidosis (fatal & non-fatal forms) or Leigh syndrome.

A set of primers (synthetic oligonucleotides) were developed that allowed simultaneous direct sequencing of mtDNA in a multiplex reaction in a single 96-well plate.

Diagnostic sequencing of mtDNA consists of the following phases: use of PCR (Polymerase Chain Reaction) to amplify 12 overlapping fragments, visualization of PCR products, sequencing using standard PCR and an automatic genetic analyser, perform PCR for sequencing in well plate, cleaning of sequence.

The first step of method to detect mutation in the human mitochondrial DNA includes use of PCR to amplify 12 overlapping fragments. Primers used in this reaction are listed in Table 1. A single PCR reaction contains the following: 5 µl of 10× *Pfu* buffer with MgSO₄, 1 µl of 10 mM dNTP mix, 50 ng/µl of DNA, 0.4 µl of *Pfu* polymerase (2.5 U/µl), 2 µl of primers (forward + reverse) (10 pmol/µl), and H₂O to reach 50 µl. Prepare the reaction mix without primers for 12 reactions in 1.5 ml tubes. 12-well strips are used to carry out all 12 reactions at the same time. 48 µl of the reaction mix and 2 µl of the primers are added to each well. The reaction conditions are described in Table 2.

**Table 1. PCR primers.**

| **Fragment** | **Primer** | **Sequence (5' - 3')** | **Fragment length** | **SEQ ID No.** |
|---|---|---|---|---|
| 1 | 14898 for^{b)} | tagccatgcactactcaccaga | 1822 | 97 |
| | 151 rev^{b)} | ggatgaggcaggaatcaaagac | | 98 |
| 2 | 16488 for^{a)} | ctgtatccgacatctggttcct | 1758 | 99 |
| | 1677 rev^{a)} | gtttagctcagagcggtcaagt | | 100 |
| 3 | 1404 for^{a)} | acttaagggtcgaaggtggatt | 1434 | 101 |
| | 2838 rev | ttgggttctgctccgaggtc | | 102 |
| 4 | 2470 for | ggaactcggcaaatcttaccc | 1477 | 103 |
| | 3947 rev^{b)} | tcgatgttgaagcctgagacta | | 104 |
| 5 | 3734 for^{b)} | aagtcaccctagccatcattcta | 1654 | 105 |
| | 5388 rev | tggggagtagtgtgattgaggtg | | 106 |
| 6 | 5140 for | gcaccacgaccctactactat | 1599 | 107 |
| | 6739 rev^{b)} | gatatcatagctcagaccatacc | | 108 |
| 7 | 6511 for^{b)} | ctgctggcatcactatactacta | 1469 | 109 |
| | 7980 rev | tcgcctggttctaggaataatgg | | 110 |
| 8 | 7661 for | cacgccctcataatcattttcc | 1559 | 111 |
| | 9220 rev^{b)} | gattggtgggtcattatgtgttg | | 112 |
| 9 | 8910 for^{a)} | cttaccacaaggcacacctaca | 1738 | 113 |
| | 10648 rev^{b)} | ggcacaatattggctaagaggg | | 114 |
| 10 | 10360 for^{b)} | gtctggcctatgagtgactaca | 1866 | 115 |
| | 12226 rev^{b)} | cagttcttgtgagctttctcgg | | 116 |
| 11 | 11976 for^{a)} | actccctctacatatttaccacaac | 1853 | 117 |
| | 13830 rev^{a)} | aagtcctaggaaagtgacagcgag | | 118 |
| 12 | 13477 for^{a)} | gcaggaatacctttcctcacag | 1872 | 119 |
| | 15349 rev^{a)} | gtgcaagaataggaggtggagt | | 120 |

| | | | | |
|---|---|---|---|---|
| ^{a)} - primers are from Torroni *et al.,* 2001 ^{b)} - primers are from Ramos *et al.,* 2009 | | | | |

**Table 2. PCR conditions.**

| | Initial denaturation | 35 cycles | | | Final extension |
|---|---|---|---|---|---|
| | | Denaturation | Annealing | Synthesis | |
| Temperature | 95°C | 95°C | 62°C | 72°C | 72°C |
| Time (sec) | 60 | 30 | 30 | 180 | 420 |

The next step is to visualize PCR products in a 1% agarose gel. 1 kb DNA Ladder was used in the following step. After that PCR products are cleaned. A single reaction for cleaning contains the following: 10 µl of PCR product, 2 µl of *Buffer Exo I,* 0.5 µl of Exo I (20 U/µl), 1 µl of FastAP (1 U/µl), and H₂O to reach 20 µl. The step includes incubation at 37°C for 15 minutes, followed by heating at 80°C for another 15 minutes.

The next step includes sequencing using standard PCR and an automatic genetic analyzer (ABI PRISM 3130x1, Applied Biosystems, Waltham, MA, USA). Four forward and four reverse sequencing primers were designed for each fragment (see Table 3). None of the primers overlap and each has an annealing temperature of 56-58°C, enabling their use in a single PCR reaction set. Reaction conditions are described in Table 4.

Later a PCR for sequencing is performed in 96-well plates. Each well contains 10 µl of reaction mix including the following: 2 µl of cleaned PCR products, 1 µl of *Big Dye,* 1 µl of 5× sequencing buffer, 2 µl of primer (5 pmol/µl), and H₂O to reach 10 µl. The reaction mix without primers or PCR products for all 96 reactions is prepared. 53 µl of reaction mix is added to each of the cleaned PCR products, and is mixed by pipetting. 8 µl of this mix and 2 µl of primer are added to each well of a 96-well plate. Reaction conditions are listed in Table 4.

**Table 3. Sequencing primers.**

| **Synthetic oligo-nucleotide** | **Localization** | **Sequence of primers** | **Length of primers** | **SEQ ID No.** |
|---|---|---|---|---|
| **1** | **2** | **3** | **4** | **5** |
| A1 | 14921 for | gcctcaaccgccttttcatc | 20 | 1 |
| B1 | 15320 for | ctagcaacactccacctcc | 19 | 2 |
| C1 | 15787 for | taccatcattggacaagtagc | 21 | 3 |
| D1 | 16202 for | aagcaagtacagcaatcaacc | 21 | 4 |
| E1 | 129 rev | agatactgcgacatagggtg | 20 | 5 |
| F1 | 16254 rev | tttggagttgcagttgatgtg | 21 | 6 |
| G1 | 15841 rev | taggattaggattgttgtgaag | 22 | 7 |
| H1 | 15362 rev | ttgatcccgtttcgtgcaag | 20 | 8 |
| A2 | 16514 for | cagggtcataaagcctaaatag | 22 | 9 |
| B2 | 362 for | caaagaaccctaacaccagc | 20 | 10 |
| C2 | 841 for | acgaaagtttaactaagctatac | 23 | 11 |
| D2 | 1203 for | cctgttctgtaatcgataaacc | 22 | 12 |
| E2 | 1652 rev | gttgaaatctcctaagtgtaag | 22 | 13 |
| F2 | 1246 rev | aagaggtggtgaggttgatc | 20 | 14 |
| G2 | 889 rev | cacgaaattgaccaaccctg | 20 | 15 |
| H2 | 409 rev | caaaagataaaatttgaaatctgg | 24 | 16 |
| A3 | 1429 for | cagtaaactaagagtagagtgc | 22 | 17 |
| B3 | 1739 for | aaagtataggcgatagaaattg | 22 | 18 |
| C3 | 2036 for | cttagttcaactttaaatttgcc | 23 | 19 |
| D3 | 2403 for | gtcattattaccctcactgtc | 21 | 20 |
| E3 | 2814 rev | caaccgaaatttttaatgcagg | 22 | 21 |
| F3 | 2450 rev | tccttatgagcatgcctgtg | 20 | 22 |
| G3 | 2100 rev | gactaacagttaaatttacaagg | 23 | 23 |
| H3 | 1792 rev | ccttgcggtactatatctattg | 22 | 24 |
| A4 | 2493 for | cctgtttaccaaaaacatcacc | 22 | 25 |
| B4 | 2821 for | cctcggagcagaacccaac | 19 | 26 |
| C4 | 3120 for | cctgtacgaaaggacaagag | 20 | 27 |
| D4 | 3487 for | ctaaaacccgccacatctac | 20 | 28 |
| E4 | 3914 rev | ccttcggcaaggtcgaagg | 19 | 29 |
| F4 | 3528 rev | ggcggtgatgtagagggtg | 19 | 30 |
| G4 | 3165 rev | ggcgctttgtgaagtaggc | 19 | 31 |
| H4 | 2861 rev | tcttagcatgtactgctcgg | 20 | 32 |
| A5 | 3758 for | tatcaacattactaataagtggc | 23 | 33 |
| B5 | 4146 for | ccgctacgaccaactcatac | 20 | 34 |
| C5 | 4502 for | taccatctttgcaggcacac | 20 | 35 |
| D5 | 4860 for | ctcacatgacaaaaactagcc | 21 | 36 |
| E5 | 5365 rev | gagtagattaggcgtaggtag | 21 | 37 |
| F5 | 4909 rev | ggagagatttggtatatgattg | 22 | 38 |
| G5 | 4550 rev | aaatcagtgcgagcttagcg | 20 | 39 |
| H5 | 4201 rev | ctagggtgagtggtaggaag | 20 | 40 |
| A6 | 5163 for | cgcacctgaaacaagctaac | 20 | 41 |
| B6 | 5521 for | ggttaaatacagaccaagagc | 21 | 42 |
| C6 | 5877 for | ctcagccattttacctcacc | 20 | 43 |
| D6 | 6304 for | ggaactactcccaccctgg | 19 | 44 |
| E6 | 6691 rev | ccggagtagtaagttacaatatg | 23 | 45 |
| F6 | 6345 rev | agatggttaggtctacggagg | 21 | 46 |
| G6 | 5919 rev | aacggtcggcgaacatcag | 19 | 47 |
| H6 | 5572 rev | taagtattgcaacttactgagg | 22 | 48 |
| A7 | 6536 for | agaccgcaacctcaacacc | 19 | 49 |
| B7 | 6848 for | caccggcgtcaaagtatttag | 21 | 50 |
| C7 | 7215 for | cgacgttactcggactacc | 19 | 51 |
| D7 | 7559 for | attataggctaaatcctatatatc | 24 | 52 |
| E7 | 7952 rev | gtatgtaggagttgaagattag | 22 | 53 |
| F7 | 7606 rev | tacttgcgctgcatgtgcc | 19 | 54 |
| G7 | 7259 rev | gatgtttcatgtggtgtatgc | 21 | 55 |
| H7 | 6888 rev | cgtggagtgtggcgagtc | 18 | 56 |
| A8 | 7689 for | gcttcctagtcctgtatgcc | 20 | 57 |
| B8 | 8046 for | ttacatcacaagacgtcttgc | 21 | 58 |
| C8 | 8462 for | taccacctacctccctcac | 19 | 59 |
| D8 | 8822 for | ctataaacctagccatggcc | 20 | 60 |
| E8 | 9196 rev | cgtgcaggtagaggcttac | 19 | 61 |
| F8 | 8865 rev | cactgtgcccgctcataag | 19 | 62 |
| G8 | 8529 rev | cattttggttctcagggtttg | 21 | 63 |
| H8 | 8103 rev | tctgtttttaagcctaatgtgg | 22 | 64 |
| A9 | 8942 for | ccatactagttattatcgaaacc | 23 | 65 |
| B9 | 9362 for | aaccatataccaatgatggcg | 21 | 66 |
| C9 | 9775 for | acggcatctacggctcaac | 19 | 67 |
| D9 | 10202 for | cgtccctttctccataaaattc | 22 | 68 |
| E9 | 10622 rev | ggtgttgagggttatgagag | 20 | 69 |
| F9 | 10248 rev | ataagaaggtaatagctactaag | 23 | 70 |
| G9 | 9819 rev | cgtggaagcctgtggctac | 19 | 71 |
| H9 | 9411 rev | cttggtatgtgctttctcgtg | 21 | 72 |
| A10 | 10387 for | gattagactgaaccgaattgg | 21 | 73 |
| B10 | 10793 for | ctaccactgacatgactttcc | 21 | 74 |
| C10 | 11279 for | ctaaacattctactactcactc | 22 | 75 |
| D10 | 11764 for | acgcactcacagtcgcatc | 19 | 76 |
| E10 | 12193 rev | tcgtaagcctctgttgtcag | 20 | 77 |
| F10 | 11808 rev | gtttgaagtccttgagagagg | 21 | 78 |
| G10 | 11328 rev | caggagtttgatagttcttgg | 21 | 79 |
| H10 | 10842 rev | gttgtgttgattcaaattatgtg | 23 | 80 |
| A11 | 12008 for | ggctcactcacccaccac | 18 | 81 |
| B11 | 12435 for | ccattatgtaaaatccattgtcg | 23 | 82 |
| C11 | 12841 for | attcaagcaatcctatacaacc | 22 | 83 |
| D11 | 13261 for | caactaggactcataatagttac | 23 | 84 |
| E11 | 13805 rev | gctgtgagttttaggtagagg | 21 | 85 |
| F11 | 13306 rev | ggtgtggttggttgatgcc | 19 | 86 |
| G11 | 12887 rev | aggatgaaaccgatatcgcc | 20 | 87 |
| H11 | 12482 rev | aagagactgataataaaggtgg | 22 | 88 |
| A12 | 13501 for | ttctactccaaagaccacatc | 21 | 89 |
| B12 | 13922 for | accctagcatcacacaccg | 19 | 90 |
| C12 | 14341 for | cccatcatactctttcaccc | 20 | 91 |
| D12 | 14752 for | cccaatacgcaaaactaaccc | 21 | 92 |
| E12 | 15323 rev | ctagggctgcaataatgaagg | 21 | 93 |
| F12 | 14810 rev | ggaggtcgatgaatgagtgg | 20 | 94 |
| G12 | 14383 rev | gatggaggtaggattggtgc | 20 | 95 |
| H12 | 13968 rev | cgtaagaaggcctagatagg | 20 | 96 |

**Table 4. Sequencing reaction conditions.**

| | 25 cycles | | |
|---|---|---|---|
| | Denaturation | Annealing | Synthesis |
| Temperature | 96°C | 56°C | 60°C |
| Time (sec) | 10 | 5 | 240 |

After sequencing said sequence is cleaned. Cleaning is performed by preparation of a gel filtration plate. Said plate is made by adding 300 µl of water to *Sephadex G-50* 1 day or ∼3 hours prior to use. If the solution is prepared on the same day, the plate is incubated at 37°C for at least 3 hours. The gel filtration plate is centrifuged at 1,500 rpm for 10 minutes. The plate is washed with 150 µl of water and centrifuged at 2,000 rpm for 8 minutes. Samples are added to the gel and centrifuged at 2,500 rpm for 8 minutes. Samples are dry filtered at 70°C for approximately 1 hour. 10 µl of formamide is added to each well and the plate heated at 95°C for 5 minutes. After heating, refrigerate the sequences immediately. Sequencing is carried out by capillary gel electrophoresis. The sequences is analysed by use of sequence analysing program, for example the *Lasergene SeqMan Pro v.7.1.0 (44.1)* program, and sequences are compared with the rCRS (*Revised Cambridge Reference Sequence*), available at http://mitomap.org/MITOMAP.

In the result a primer sequences were developed for the sequencing reaction.

Developed test kit comprises 96 primers for the uniform sequencing reaction with a single melting temperature of 56°C. This feature enables a robust reaction setup, and allows for automatization or semi-automatization of the process. Subsequent sequencing of PCR products with 96 oligonucleotides allows 4-6-fold coverage of the mtDNA genome.

To validate the kit 56 mtDNA samples were analysed using developed sequencing primers. Seven samples were controls from healthy individuals, and 49 were from patients with symptoms of (suspected) mitochondrial disease. In two patients' samples, mitochondrial disease was confirmed, as was the diagnosis of Leigh syndrome. We identified the mutations mt.G13513R (p.D393N) in the *mt-ND5* gene and mt.9185T>C (p.L220P) in the *mt-ATP6* gene.

We developed the sequencing primer set to improve the healthcare of mitochondrial disease patients. Application of this test will minimize the need for invasive muscle biopsy and hospitalization of patients with mitochondrial disease.

The sequencing primer set is suited for the diagnostics of mitochondrial pathology caused by mtDNA mutations. Its main application is as a clinical test to confirm the presence of a mutation in a patient sample.

The sequencing primer set can be used in all diagnostic laboratories with various levels of process automatization.

## Claims

1. A kit for detecting mutation or polymorphism in the human mitochondrial DNA **characterized in that** the kit comprises the 96 sequencing primers of SEQ ID Nos. 1-96.

## Patentansprüche

1. Ein Kit zum Nachweis von Mutation oder von Polymorphismus in Mitochondialer DNA, **dadurch gekennzeichnet, dass** ein Kit 96 Sequenzier-Primern von SEQ ID NOS: 1-96 umfasst.

## Revendications

1. Kit de détection d'une mutation ou d'un polymorphisme dans l'adn mitochondrial human, **caractérisée en ce que** kit comprend des 96 amorces de séquençage de SEQ ID NO: 1-96.


### SEQUENCE LISTING

<110> Latvian Biomedical Research and Study Centre
<120> A set for detecting mutation or polymorphism in the human mitochondrial DNA
<130> AK/P3040
<160> 120
<170> BiSSAP 1.3
<210> 1
   <211> 20
   <212> DNA
   <213> Artificial Sequence
<220>
   <223> Primer Sequence
<400> 1
   gcctcaaccg ccttttcatc 20
<210> 2
   <211> 19
   <212> DNA
   <213> Artificial Sequence
<220>
   <223> Primer Sequence
<400> 2
   ctagcaacac tccacctcc 19
<210> 3
   <211> 21
   <212> DNA
   <213> Artificial Sequence
<220>
   <223> Primer Sequence
<400> 3
   taccatcatt ggacaagtag c 21
<210> 4
   <211> 21
   <212> DNA
   <213> Artificial Sequence
<220>
   <223> Primer Sequence
<400> 4
   aagcaagtac agcaatcaac c 21
<210> 5
   <211> 20
   <212> DNA
   <213> Artificial Sequence
<220>
   <223> Primer Sequence
<400> 5
   agatactgcg acatagggtg 20
<210> 6
   <211> 21
   <212> DNA
   <213> Artificial Sequence
<220>
   <223> Primer Sequence
<400> 6
   tttggagttg cagttgatgt g 21
<210> 7
   <211> 22
   <212> DNA
   <213> Artificial Sequence
<220>
   <223> Primer Sequence
<400> 7
   taggattagg attgttgtga ag 22
<210> 8
   <211> 20
   <212> DNA
   <213> Artificial Sequence
<220>
   <223> Primer Sequence
<400> 8
   ttgatcccgt ttcgtgcaag 20
<210> 9
   <211> 22
   <212> DNA
   <213> Artificial Sequence
<220>
   <223> Primer Sequence
<400> 9
   cagggtcata aagcctaaat ag 22
<210> 10
   <211> 20
   <212> DNA
   <213> Artificial Sequence
<220>
   <223> Primer Sequence
<400> 10
   caaagaaccc taacaccagc 20
<210> 11
   <211> 23
   <212> DNA
   <213> Artificial Sequence
<220>
   <223> Primer Sequnce
<400> 11
   acgaaagttt aactaagcta tac 23
<210> 12
   <211> 22
   <212> DNA
   <213> Artificial Sequence
<220>
   <223> Primer Sequence
<400> 12
   cctgttctgt aatcgataaa cc 22
<210> 13
   <211> 22
   <212> DNA
   <213> Artificial Sequence
<220>
   <223> Primer Sequence
<400> 13
   gttgaaatct cctaagtgta ag 22
<210> 14
   <211> 20
   <212> DNA
   <213> Artificial Sequence
<220>
   <223> Primer Sequence
<400> 14
   aagaggtggt gaggttgatc 20
<210> 15
   <211> 20
   <212> DNA
   <213> Artificial Sequence
<220>
   <223> Primer Sequence
<400> 15
   cacgaaattg accaaccctg 20
<210> 16
   <211> 24
   <212> DNA
   <213> Artificial Sequence
<220>
   <223> Primer Sequence
<400> 16
   caaaagataa aatttgaaat ctgg 24
<210> 17
   <211> 22
   <212> DNA
   <213> Artificial Sequence
<220>
   <223> Primer Sequence
<400> 17
   cagtaaacta agagtagagt gc 22
<210> 18
   <211> 22
   <212> DNA
   <213> Artificial Sequence
<220>
   <223> Primer Sequence
<400> 18
   aaagtatagg cgatagaaat tg 22
<210> 19
   <211> 23
   <212> DNA
   <213> Artificial Sequence
<220>
   <223> Primer Sequence
<400> 19
   cttagttcaa ctttaaattt gcc 23
<210> 20
   <211> 21
   <212> DNA
   <213> Artificial Sequence
<220>
   <223> Primer Sequence
<400> 20
   gtcattatta ccctcactgt c 21
<210> 21
   <211> 22
   <212> DNA
   <213> Artificial Sequence
<220>
   <223> Primer Sequence
<400> 21
   caaccgaaat ttttaatgca gg 22
<210> 22
   <211> 20
   <212> DNA
   <213> Artificial Sequence
<220>
   <223> Primer Sequence
<400> 22
   tccttatgag catgcctgtg 20
<210> 23
   <211> 23
   <212> DNA
   <213> Artificial Sequence
<220>
   <223> Primer Sequence
<400> 23
   gactaacagt taaatttaca agg 23
<210> 24
   <211> 22
   <212> DNA
   <213> Artificial Sequence
<220>
   <223> Primer Sequence
<400> 24
   ccttgcggta ctatatctat tg 22
<210> 25
   <211> 22
   <212> DNA
   <213> Artificial Sequence
<220>
   <223> Primer Sequence
<400> 25
   cctgtttacc aaaaacatca cc 22
<210> 26
   <211> 19
   <212> DNA
   <213> Artificial Sequence
<220>
   <223> Primer Sequence
<400> 26
   cctcggagca gaacccaac 19
<210> 27
   <211> 20
   <212> DNA
   <213> Artificial Sequence
<220>
   <223> Primer Sequence
<400> 27
   cctgtacgaa aggacaagag 20
<210> 28
   <211> 20
   <212> DNA
   <213> Artificial Sequence
<220>
   <223> Primer Sequence
<400> 28
   ctaaaacccg ccacatctac 20
<210> 29
   <211> 19
   <212> DNA
   <213> Artificial Sequence
<220>
   <223> Primer Sequence
<400> 29
   ccttcggcaa ggtcgaagg 19
<210> 30
   <211> 19
   <212> DNA
   <213> Artificial Sequence
<220>
   <223> Primer Sequence
<400> 30
   ggcggtgatg tagagggtg 19
<210> 31
   <211> 19
   <212> DNA
   <213> Artificial Sequence
<220>
   <223> Primer Sequence
<400> 31
   ggcgctttgt gaagtaggc 19
<210> 32
   <211> 20
   <212> DNA
   <213> Artificial Sequence
<220>
   <223> Primer Sequence
<400> 32
   tcttagcatg tactgctcgg 20
<210> 33
   <211> 23
   <212> DNA
   <213> Artificial Sequence
<220>
   <223> Primer Sequence
<400> 33
   tatcaacatt actaataagt ggc 23
<210> 34
   <211> 20
   <212> DNA
   <213> Artificial Sequence
<220>
   <223> Primer Sequence
<400> 34
   ccgctacgac caactcatac 20
<210> 35
   <211> 20
   <212> DNA
   <213> Artificial Sequence
<220>
   <223> Primer Sequence
<400> 35
   taccatcttt gcaggcacac 20
<210> 36
   <211> 21
   <212> DNA
   <213> Artificial Sequence
<220>
   <223> Primer Sequence
<400> 36
   ctcacatgac aaaaactagc c 21
<210> 37
   <211> 21
   <212> DNA
   <213> Artificial Sequence
<220>
   <223> Primer Sequence
<400> 37
   gagtagatta ggcgtaggta g 21
<210> 38
   <211> 22
   <212> DNA
   <213> Artificial Sequence
<220>
   <223> Primer Sequence
<400> 38
   ggagagattt ggtatatgat tg 22
<210> 39
   <211> 20
   <212> DNA
   <213> Artificial Sequence
<220>
   <223> Primer Sequence
<400> 39
   aaatcagtgc gagcttagcg 20
<210> 40
   <211> 20
   <212> DNA
   <213> Artificial Sequence
<220>
   <223> Primer Sequence
<400> 40
   ctagggtgag tggtaggaag 20
<210> 41
   <211> 20
   <212> DNA
   <213> Artificial Sequence
<220>
   <223> Primer Sequence
<400> 41
   cgcacctgaa acaagctaac 20
<210> 42
   <211> 21
   <212> DNA
   <213> Artificial Sequence
<220>
   <223> Primer Sequence
<400> 42
   ggttaaatac agaccaagag c 21
<210> 43
   <211> 20
   <212> DNA
   <213> Artificial Sequence
<220>
   <223> Primer Sequence
<400> 43
   ctcagccatt ttacctcacc 20
<210> 44
   <211> 19
   <212> DNA
   <213> Artificial Sequence
<220>
   <223> Primer Sequence
<400> 44
   ggaactactc ccaccctgg 19
<210> 45
   <211> 23
   <212> DNA
   <213> Artificial Sequence
<220>
   <223> Primer Sequence
<400> 45
   ccggagtagt aagttacaat atg 23
<210> 46
   <211> 21
   <212> DNA
   <213> Artificial Sequence
<220>
   <223> Primer Sequence
<400> 46
   agatggttag gtctacggag g 21
<210> 47
   <211> 19
   <212> DNA
   <213> Artificial Sequence
<220>
   <223> Primer Sequence
<400> 47
   aacggtcggc gaacatcag 19
<210> 48
   <211> 22
   <212> DNA
   <213> Artificial Sequence
<220>
   <223> Primer Sequence
<400> 48
   taagtattgc aacttactga gg 22
<210> 49
   <211> 19
   <212> DNA
   <213> Artificial Sequence
<220>
   <223> Primer Sequence
<400> 49
   agaccgcaac ctcaacacc 19
<210> 50
   <211> 21
   <212> DNA
   <213> Artificial Sequence
<220>
   <223> Primer Sequence
<400> 50
   caccggcgtc aaagtattta g 21
<210> 51
   <211> 19
   <212> DNA
   <213> Artificial Sequence
<220>
   <223> Primer Sequence
<400> 51
   cgacgttact cggactacc 19
<210> 52
   <211> 24
   <212> DNA
   <213> Artificial Sequence
<220>
   <223> Primer Sequence
<400> 52
   attataggct aaatcctata tatc 24
<210> 53
   <211> 22
   <212> DNA
   <213> Artificial Sequence
<220>
   <223> Primer Sequence
<400> 53
   gtatgtagga gttgaagatt ag 22
<210> 54
   <211> 19
   <212> DNA
   <213> Artificial Sequence
<220>
   <223> Primer Sequence
<400> 54
   tacttgcgct gcatgtgcc 19
<210> 55
   <211> 21
   <212> DNA
   <213> Artificial Sequence
<220>
   <223> Primer Sequence
<400> 55
   gatgtttcat gtggtgtatg c 21
<210> 56
   <211> 18
   <212> DNA
   <213> Artificial Sequence
<220>
   <223> Primer Sequence
<400> 56
   cgtggagtgt ggcgagtc 18
<210> 57
   <211> 20
   <212> DNA
   <213> Artificial Sequence
<220>
   <223> Primer Sequence
<400> 57
   gcttcctagt cctgtatgcc 20
<210> 58
   <211> 21
   <212> DNA
   <213> Artificial Sequence
<220>
   <223> Primer Sequence
<400> 58
   ttacatcaca agacgtcttg c 21
<210> 59
   <211> 19
   <212> DNA
   <213> Artificial Sequence
<220>
   <223> Primer Sequence
<400> 59
   taccacctac ctccctcac 19
<210> 60
   <211> 20
   <212> DNA
   <213> Artificial Sequence
<220>
   <223> Primer Sequence
<400> 60
   ctataaacct agccatggcc 20
<210> 61
   <211> 19
   <212> DNA
   <213> Artificial Sequence
<220>
   <223> Primer Sequence
<400> 61
   cgtgcaggta gaggcttac 19
<210> 62
   <211> 19
   <212> DNA
   <213> Artificial Sequence
<220>
   <223> Primer Sequence
<400> 62
   cactgtgccc gctcataag 19
<210> 63
   <211> 21
   <212> DNA
   <213> Artificial Sequence
<220>
   <223> Primer Sequence
<400> 63
   cattttggtt ctcagggttt g 21
<210> 64
   <211> 22
   <212> DNA
   <213> Artificial Sequence
<220>
   <223> Primer Sequence
<400> 64
   tctgttttta agcctaatgt gg 22
<210> 65
   <211> 23
   <212> DNA
   <213> Artificial Sequence
<220>
   <223> Primer Sequence
<400> 65
   ccatactagt tattatcgaa acc 23
<210> 66
   <211> 21
   <212> DNA
   <213> Artificial Sequence
<220>
   <223> Primer Sequence
<400> 66
   aaccatatac caatgatggc g 21
<210> 67
   <211> 19
   <212> DNA
   <213> Artificial Sequence
<220>
   <223> Primer Sequence
<400> 67
   acggcatcta cggctcaac 19
<210> 68
   <211> 22
   <212> DNA
   <213> Artificial Sequence
<220>
   <223> Primer Sequence
<400> 68
   cgtccctttc tccataaaat tc 22
<210> 69
   <211> 20
   <212> DNA
   <213> Artificial Sequence
<220>
   <223> Primer Sequence
<400> 69
   ggtgttgagg gttatgagag 20
<210> 70
   <211> 23
   <212> DNA
   <213> Artificial Sequence
<220>
   <223> Primer Sequence
<400> 70
   ataagaaggt aatagctact aag 23
<210> 71
   <211> 19
   <212> DNA
   <213> Artificial Sequence
<220>
   <223> Primer Sequence
<400> 71
   cgtggaagcc tgtggctac 19
<210> 72
   <211> 21
   <212> DNA
   <213> Artificial Sequence
<220>
   <223> Primer Sequence
<400> 72
   cttggtatgt gctttctcgt g 21
<210> 73
   <211> 21
   <212> DNA
   <213> Artificial Sequence
<220>
   <223> Primer Sequence
<400> 73
   gattagactg aaccgaattg g 21
<210> 74
   <211> 21
   <212> DNA
   <213> Artificial Sequence
<220>
   <223> Primer Sequence
<400> 74
   ctaccactga catgactttc c 21
<210> 75
   <211> 22
   <212> DNA
   <213> Artificial Sequence
<220>
   <223> Primer Sequence
<400> 75
   ctaaacattc tactactcac tc 22
<210> 76
   <211> 19
   <212> DNA
   <213> Artificial Sequence
<220>
   <223> Primer Sequence
<400> 76
   acgcactcac agtcgcatc 19
<210> 77
   <211> 20
   <212> DNA
   <213> Artificial Sequence
<220>
   <223> Primer Sequence
<400> 77
   tcgtaagcct ctgttgtcag 20
<210> 78
   <211> 21
   <212> DNA
   <213> Artificial Sequence
<220>
   <223> Primer Sequence
<400> 78
   gtttgaagtc cttgagagag g 21
<210> 79
   <211> 21
   <212> DNA
   <213> Artificial Sequence
<220>
   <223> Primer Sequence
<400> 79
   caggagtttg atagttcttg g 21
<210> 80
   <211> 23
   <212> DNA
   <213> Artificial Sequence
<220>
   <223> Primer Sequence
<400> 80
   gttgtgttga ttcaaattat gtg 23
<210> 81
   <211> 18
   <212> DNA
   <213> Artificial Sequence
<220>
   <223> Primer Sequence
<400> 81
   ggctcactca cccaccac 18
<210> 82
   <211> 23
   <212> DNA
   <213> Artificial Sequence
<220>
   <223> Primer Sequence
<400> 82
   ccattatgta aaatccattg tcg 23
<210> 83
   <211> 22
   <212> DNA
   <213> Artificial Sequence
<220>
   <223> Primer Sequence
<400> 83
   attcaagcaa tcctatacaa cc 22
<210> 84
   <211> 23
   <212> DNA
   <213> Artificial Sequence
<220>
   <223> Primer Sequence
<400> 84
   caactaggac tcataatagt tac 23
<210> 85
   <211> 21
   <212> DNA
   <213> Artificial Sequence
<220>
   <223> Primer Sequence
<400> 85
   gctgtgagtt ttaggtagag g 21
<210> 86
   <211> 19
   <212> DNA
   <213> Artificial Sequence
<220>
   <223> Primer Sequence
<400> 86
   ggtgtggttg gttgatgcc 19
<210> 87
   <211> 20
   <212> DNA
   <213> Artificial Sequence
<220>
   <223> Primer Sequence
<400> 87
   aggatgaaac cgatatcgcc 20
<210> 88
   <211> 22
   <212> DNA
   <213> Artificial Sequence
<220>
   <223> Primer Sequence
<400> 88
   aagagactga taataaaggt gg 22
<210> 89
   <211> 21
   <212> DNA
   <213> Artificial Sequence
<220>
   <223> Primer Sequence
<400> 89
   ttctactcca aagaccacat c 21
<210> 90
   <211> 19
   <212> DNA
   <213> Artificial Sequence
<220>
   <223> Primer Sequence
<400> 90
   accctagcat cacacaccg 19
<210> 91
   <211> 20
   <212> DNA
   <213> Artificial Sequence
<220>
   <223> Primer Sequence
<400> 91
   cccatcatac tctttcaccc 20
<210> 92
   <211> 21
   <212> DNA
   <213> Artificial Sequence
<220>
   <223> Primer Sequence
<400> 92
   cccaatacgc aaaactaacc c 21
<210> 93
   <211> 21
   <212> DNA
   <213> Artificial Sequence
<220>
   <223> Primer Sequence
<400> 93
   ctagggctgc aataatgaag g 21
<210> 94
   <211> 20
   <212> DNA
   <213> Artificial Sequence
<220>
   <223> Primer Sequence
<400> 94
   ggaggtcgat gaatgagtgg 20
<210> 95
   <211> 20
   <212> DNA
   <213> Artificial Sequence
<220>
   <223> Pimer Sequence
<400> 95
   gatggaggta ggattggtgc 20
<210> 96
   <211> 20
   <212> DNA
   <213> Artificial Sequence
<220>
   <223> Primer Sequence
<400> 96
   cgtaagaagg cctagatagg 20
<210> 97
   <211> 22
   <212> DNA
   <213> Artificial Sequence
<220>
   <223> Primer Sequence
<400> 97
   tagccatgca ctactcacca ga 22
<210> 98
   <211> 22
   <212> DNA
   <213> Artificial Sequence
<220>
   <223> Primer Sequence
<400> 98
   ggatgaggca ggaatcaaag ac 22
<210> 99
   <211> 22
   <212> DNA
   <213> Artificial Sequence
<220>
   <223> Primer Sequence
<400> 99
   ctgtatccga catctggttc ct 22
<210> 100
   <211> 22
   <212> DNA
   <213> Artificial Sequence
<220>
   <223> Primer Sequence
<400> 100
   gtttagctca gagcggtcaa gt 22
<210> 101
   <211> 22
   <212> DNA
   <213> Artificial Sequence
<220>
   <223> Primer Sequence
<400> 101
   acttaagggt cgaaggtgga tt 22
<210> 102
   <211> 20
   <212> DNA
   <213> Artificial Sequence
<220>
   <223> Primer Sequence
<400> 102
   ttgggttctg ctccgaggtc 20
<210> 103
   <211> 21
   <212> DNA
   <213> Artificial Sequence
<220>
   <223> Primer Sequence
<400> 103
   ggaactcggc aaatcttacc c 21
<210> 104
   <211> 22
   <212> DNA
   <213> Artificial Sequence
<220>
   <223> Primer Sequence
<400> 104
   tcgatgttga agcctgagac ta 22
<210> 105
   <211> 23
   <212> DNA
   <213> Artificial Sequence
<220>
   <223> Primer Sequence
<400> 105
   aagtcaccct agccatcatt cta 23
<210> 106
   <211> 23
   <212> DNA
   <213> Artificial Sequence
<220>
   <223> Prmer Sequence
<400> 106
   tggggagtag tgtgattgag gtg 23
<210> 107
   <211> 21
   <212> DNA
   <213> Artificial Sequence
<220>
   <223> Primer Sequence
<400> 107
   gcaccacgac cctactacta t 21
<210> 108
   <211> 23
   <212> DNA
   <213> Artificial Sequence
<220>
   <223> Primer Sequence
<400> 108
   gatatcatag ctcagaccat acc 23
<210> 109
   <211> 23
   <212> DNA
   <213> Artificial Sequence
<220>
   <223> Primer Sequence
<400> 109
   ctgctggcat cactatacta cta 23
<210> 110
   <211> 23
   <212> DNA
   <213> Artificial Sequence
<220>
   <223> Primer Sequence
<400> 110
   tcgcctggtt ctaggaataa tgg 23
<210> 111
   <211> 22
   <212> DNA
   <213> Artificial Sequence
<220>
   <223> Primer Sequence
<400> 111
   cacgccctca taatcatttt cc 22
<210> 112
   <211> 23
   <212> DNA
   <213> Artificial Sequence
<220>
   <223> Primer Sequence
<400> 112
   gattggtggg tcattatgtg ttg 23
<210> 113
   <211> 22
   <212> DNA
   <213> Artificial Sequence
<220>
   <223> Primer Sequence
<400> 113
   cttaccacaa ggcacaccta ca 22
<210> 114
   <211> 22
   <212> DNA
   <213> Artificial Sequence
<220>
   <223> Primer Sequence
<400> 114
   ggcacaatat tggctaagag gg 22
<210> 115
   <211> 22
   <212> DNA
   <213> Artificial Sequence
<220>
   <223> Primer Sequence
<400> 115
   gtctggccta tgagtgacta ca 22
<210> 116
   <211> 22
   <212> DNA
   <213> Artificial Sequence
<220>
   <223> Primer Sequence
<400> 116
   cagttcttgt gagctttctc gg 22
<210> 117
   <211> 25
   <212> DNA
   <213> Artificial Sequence
<220>
   <223> Primer Sequence
<400> 117
   actccctcta catatttacc acaac 25
<210> 118
   <211> 24
   <212> DNA
   <213> Artificial Sequence
<220>
   <223> Primer Sequence
<400> 118
   aagtcctagg aaagtgacag cgag 24
<210> 119
   <211> 22
   <212> DNA
   <213> Artificial Sequence
<220>
   <223> Primer Sequence
<400> 119
   gcaggaatac ctttcctcac ag 22
<210> 120
   <211> 22
   <212> DNA
   <213> Artificial Sequence
<220>
   <223> Primer Sequence
<400> 120
   gtgcaagaat aggaggtgga gt 22